# EUROPÄISCHE PATENTANMELDUNG

(11) **EP 4 588 925 A1**
(43) Veröffentlichungstag der Anmeldung: **23.07.2025**
(21) Anmeldenummer: 24153127.6
(22) Anmeldetag: 22.01.2024
(51) Int. Cl.: C07F 9/58

(54) **ALKYL-PICOLYLLIGANDEN UND DEREN EINSATZ IN DER ALKOXYCARBONYLIERUNG**

(71) Anmelder: Evonik Oxeno GmbH & Co. KG, 45772 Marl (DE)
(72) Erfinder: MÜLLER, Stefan, 45659 Recklinghausen (DE); KUCMIERCZYK, Peter, 44628 Herne (DE); FRIDAG, Dirk, 45721 Haltern am See (DE); BÜKER, Julia, 44787 Bochum (DE); MARKOVIC, Ana, 45721 Haltern am See (DE); PESCHKE, Roland, 47057 Duisburg (DE)
(74) Vertreter: Evonik Patent Association

(57) **Zusammenfassung**

Alkyl-Picolylliganden und deren Einsatz in der Alkoxycarbonylierung.

## Beschreibung

Die Erfindung betrifft Alkyl-Picolylliganden und deren Einsatz in der Alkoxycarbonylierung.

Die Alkoxycarbonylierung ethylenisch ungesättigter Verbindungen ist ein Prozess mit steigender Bedeutung. Unter einer Alkoxycarbonylierung versteht man die Umsetzung ethylenisch ungesättigter Verbindungen (Olefinen) mit Kohlenmonoxid und Alkoholen in Gegenwart eines Metall-Ligand-Komplexes zu den entsprechenden Estern. Üblicherweise wird als Metall Palladium eingesetzt. Das folgende Schema zeigt die allgemeine Reaktionsgleichung einer Alkoxycarbonylierung:

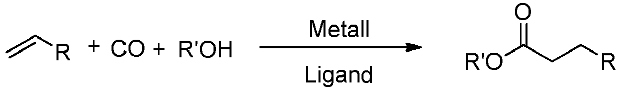

Die technische Aufgabe der Erfindung ist die Bereitstellung neuer Liganden bzw. eines neuen Verfahrens, mit welchem die Ausbeute an Ester gesteigert werden kann.

Die Aufgabe wird gelöst durch eine Verbindung nach Anspruch 1.

Verbindung, welche die Struktur **(1)** oder **(2)** aufweist:

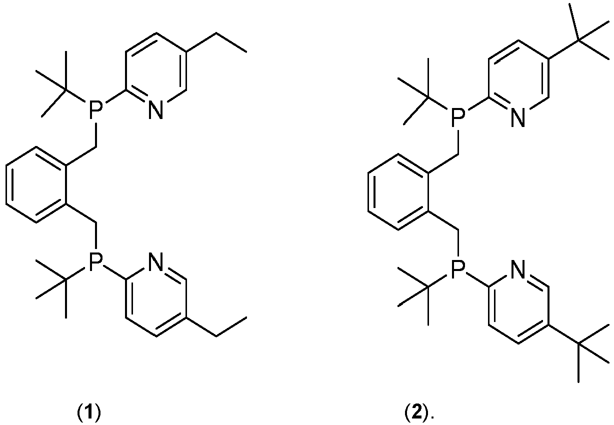

In einer Ausführungsform weist die Verbindung die Struktur **(1)** auf:

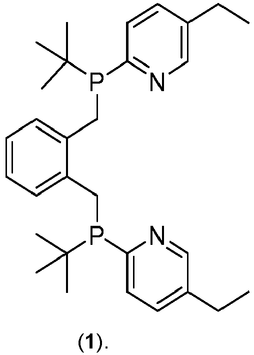

In einer Ausführungsform weist die Verbindung die Struktur **(2)** auf:

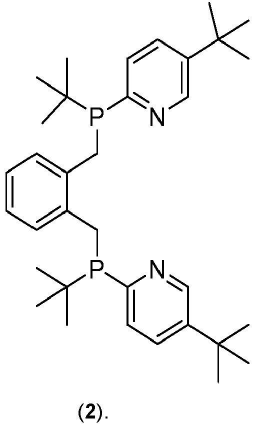

Neben den Verbindungen an sich, wird auch ein Verfahren beansprucht, in welchem die Verbindungen zum Einsatz kommen.

Verfahren umfassend die Verfahrensschritte:
a) Vorlegen einer ethylenisch ungesättigten Verbindung oder eines Gemisches aus ethylenisch ungesättigten Verbindungen;
b) Zugabe einer Verbindung, welche die Struktur **(1)** oder **(2)** aufweist: 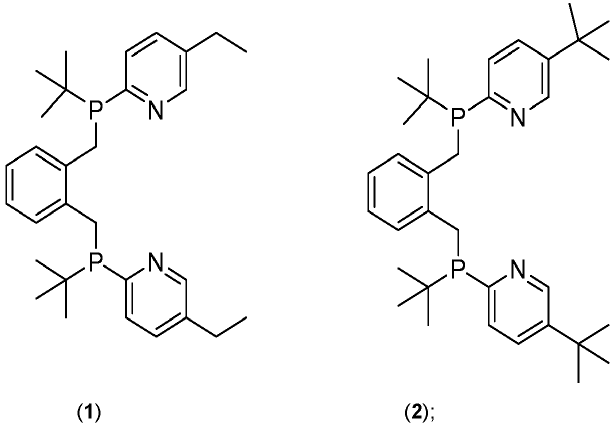
c) Zugabe einer Pd-Verbindung;
d) Zugabe eines Co-Katalysators ausgewählt aus: Aluminiumtriflat, H₂SO₄, MSA, pTSA, TFA;
e) Zugabe eines Alkohols;
f) Zuführen von CO;
g) Erwärmen des Reaktionsgemisches aus a) bis f), wobei die ethylenisch ungesättigte Verbindung zu einem Ester umgesetzt wird.

In einer Variante des Verfahrens ist die Pd-Verbindung ausgewählt aus: Palladiumdichlorid, Palladium(II)-acetylacetonat, Palladium(II)-acetat, Dichloro(1,5-cyclooctadiene)palladium(II),
Bis(dibenzylideneaceton)palladium, Bis(acetonitrile)dichloropalladium(II), Palladium(cinnamyl)dichlorid.

In einer Variante des Verfahrens ist die Pd-Verbindung Pd(acac)₂.

In einer Variante des Verfahrens ist der Co-Katalysators Aluminiumtriflat.

In einer Variante des Verfahrens ist der Alkohol in Verfahrensschritt e) ausgewählt aus: Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

In einer Variante des Verfahrens ist der Alkohol in Verfahrensschritt e) Methanol.

In einer Variante des Verfahrens wird CO in Verfahrensschritt f) mit einem Druck im Bereich von 1 bis 5 MPa (10 bis 50 bar) zugeführt.

In einer Variante des Verfahrens wird CO in Verfahrensschritt f) mit einem Druck im Bereich von 1 bis 3 MPa (10 bis 30 bar) zugeführt.

In einer Variante des Verfahrens wird das Reaktionsgemisch in Verfahrensschritt g) auf eine Temperatur im Bereich von 80 °C bis 160 °C erwärmt.

In einer Variante des Verfahrens wird das Reaktionsgemisch in Verfahrensschritt g) auf eine Temperatur im Bereich von 100 °C bis 140 °C erwärmt.

In einer Variante des Verfahrens wird in Verfahrensschritt a) ein Gemisch aus ethylenisch ungesättigten Verbindungen vorgelegt.

In einer Variante des Verfahrens wird in Verfahrensschritt a) ein Gemisch aus n-Octenen vorgelegt.

Im Folgenden soll die Erfindung anhand von Ausführungsbeispielen näher erläutert werden.

Die Reaktion wird in 20 mL Glasgefäßen mit Magnetrührfischen durchgeführt. Zunächst werden Al(OTf)₃ (9,5 mg, 0.2 mol%) und der Ligand (0.1 mol%) in dem Glasgefäß eingewogen und daraufhin luftdicht mittels eines gebördelten Septums verschlossen. Mittels durchstochener Kanüle, welche an eine Argon-Verteilerstation angeschlossen ist, wird in den folgenden Schritten eine Argonatmosphäre garantiert und besteht gleichzeitig die Möglichkeit des Druckausgleichs. Die benötigte Menge an Präkursorstammlösung (0,8 mL) wird mittels µL-Spritze zugegeben, sodass sich eine Einwaage von Pd(acac)₂ (0,8 mg, 0,025 mol%) ergibt. Dann erfolgt die Zugabe von 0,3 mL Ethylbenzol als interner Standard. Anschließend wird Methanol mittels µL-Spritze hinzugegeben, damit ein Gesamtvolumen von 8,4 mL vorliegt. Der Autoklav wird verschlossen und dreimal mit Stickstoff gespült. Danach wird CO mit einem Druck von 20 bar zugeführt. Die Reaktionslösung wird dann auf die benötigte Temperatur von 120°C erhitzt. Das Substrat (ein n-Octengemisch) wird zudosiert. Nach 15 min wird eine Probe gezogen.

Das eingesetzte n-Octengemisch bestand aus den C8-Isomeren: 1-Octen, cis-2-Octen, trans-2-Octen, cis-3-Octen, trans-3-Octen, cis-4-Octen und trans-4-Octen.

Die Reaktion wurde mit den Liganden (1), (2) und (3) durchgeführt. Der Versuch mit Ligand (3) dient als Vergleichsversuch.

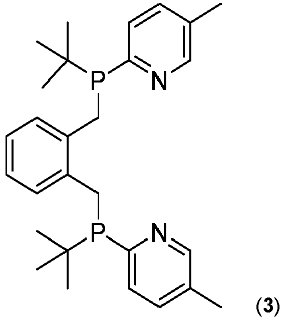

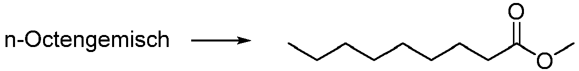

### Reaktionsbedingungen:

Pd(acac)₂, Ligand (X), Al(OTf)₃, MeOH, CO: 20 bar, 120 °C, 15 min.

Die Ausbeuten an Ester sind in der nachfolgenden Tabelle aufgelistet:

| Ligand | Ausbeute |
|---|---|
| (1)* | 62% |
| (2)* | 57% |
| (3) | 55% |

| | |
|---|---|
| * erfindungsgemäßes Ausführungsbeispiel | |

Die durchgeführten Versuche belegen, dass die gestellte Aufgabe durch eine erfindungsgemäße Verbindung gelöst wird.

## Patentansprüche

1. Verbindung, welche die Struktur (1) oder (2) aufweist:

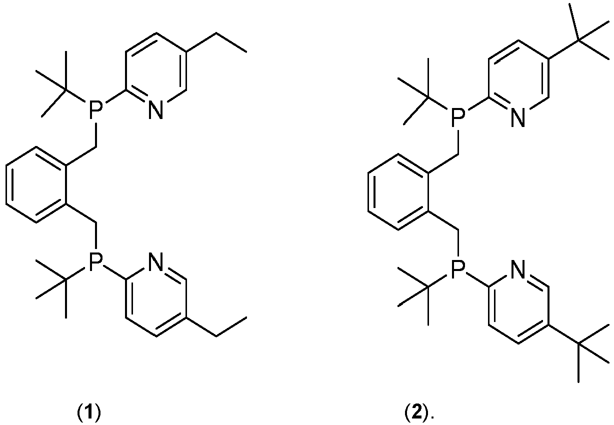

2. Verbindung nach Anspruch 1, welche die Struktur (1) aufweist:

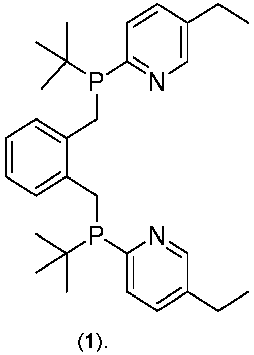

3. Verbindung nach Anspruch 1, welche die Struktur (2) aufweist:

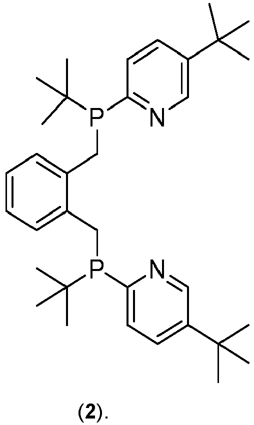

4. Verfahren umfassend die Verfahrensschritte:
a) Vorlegen einer ethylenisch ungesättigten Verbindung oder eines Gemisches aus ethylenisch ungesättigten Verbindungen;
b) Zugabe einer Verbindung, welche die Struktur **(1)** oder **(2)** aufweist: 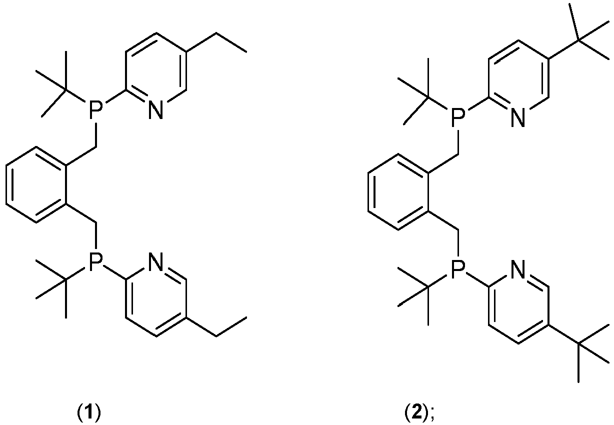
c) Zugabe einer Pd-Verbindung;
d) Zugabe eines Co-Katalysators ausgewählt aus: Aluminiumtriflat, H₂SO₄, MSA, pTSA, TFA;
e) Zugabe eines Alkohols;
f) Zuführen von CO;
g) Erwärmen des Reaktionsgemisches aus a) bis f), wobei die ethylenisch ungesättigte Verbindung zu einem Ester umgesetzt wird.

5. Verfahren nach Anspruch 4,
wobei die Pd-Verbindung ausgewählt ist aus: Palladiumdichlorid, Palladium(II)-acetylacetonat, Palladium(II)-acetat, Dichloro(1,5-cyclooctadiene)palladium(II), Bis(dibenzylideneaceton)palladium, Bis(acetonitrile)dichloropalladium(II), Palladium(cinnamyl)dichlorid.

6. Verfahren nach einem der Ansprüche 4 oder 5,
wobei die Pd-Verbindung Pd(acac)₂ ist.

7. Verfahren nach einem der Ansprüche 4 bis 6,
wobei der Co-Katalysators Aluminiumtriflat ist.

8. Verfahren nach einem der Ansprüche 4 bis 7,
wobei der Alkohol in Verfahrensschritt e) ausgewählt ist aus: Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

9. Verfahren nach einem der Ansprüche 4 bis 8,
wobei der Alkohol in Verfahrensschritt e) Methanol ist.

10. Verfahren nach einem der Ansprüche 4 bis 9,
wobei CO in Verfahrensschritt f) mit einem Druck im Bereich von 1 bis 5 MPa (10 bis 50 bar) zugeführt wird.

11. Verfahren nach einem der Ansprüche 4 bis 10,
wobei das Reaktionsgemisch in Verfahrensschritt g) auf eine Temperatur im Bereich von 80 °C bis 160 °C erwärmt wird.

12. Verfahren nach einem der Ansprüche 4 bis 11,
wobei in Verfahrensschritt a) ein Gemisch aus ethylenisch ungesättigten Verbindungen vorgelegt wird.

13. Verfahren nach einem der Ansprüche 4 bis 12,
wobei in Verfahrensschritt a) ein Gemisch aus n-Octenen vorgelegt wird.
